# EUROPEAN PATENT SPECIFICATION

(11) **EP 2 349 243 B1**
(45) Date of publication and mention of the grant of the patent: **11.11.2015**
(21) Application number: 09759912.0
(22) Date of filing: 21.10.2009
(51) Int. Cl.: A61K 31/17, A61P 17/00, A61K 47/32, A61K 9/00, A61K 9/70

(54) **UREA-BASED FILM-FORMING SOLUTION FOR TREATING NAIL PSORIASIS**
FILMBILDENDE LÖSUNG AUF HARNSTOFFBASIS ZUR BEHANDLUNG VON PSORIASIS DES NAGELS
SOLUTION À BASE D'URÉE FORMANT UN FILM POUR LE TRAITEMENT DU PSORIASIS DES ONGLES

(30) Priority: 21.10.2008 FR 0857146
(43) Date of publication of application: 03.08.2011
(73) Proprietor: Pierre Fabre Dermo-Cosmétique, 92100 Boulogne-Billancourt (FR)
(72) Inventor: CHESNOY, Sophie, F-59000 Lille (FR); DELAUNOIS, Marlène, F-31290 Cessales (FR); COUBETERGUES, Héla, F-31240 Saint-Jean (FR); LEFRANCOIS, Pascal, F-81500 Lavaur (FR)
(74) Representative: Regimbeau
(86) International application number: PCT/EP2009/063771
(87) International publication number: WO 2010/046375

(56) References cited:
- WO-A1-02/098380
- WO-A1-2005/034956
- WO-A2-2005/011565
- FR-A1- 2 613 227
- KRÜGER N ET AL: "Effect of urea nail lacquer on nail quality; Clinical evaluation and biophysical measurements" DER HAUTARZT ; ZEITSCHRIFT FÜR DERMATOLOGIE, VENEROLOGIE UND VERWANDTE GEBIETE, SPRINGER, BERLIN, DE, vol. 57, no. 12, 1 December 2006 (2006-12-01), pages 1089-1094, XP019446849 ISSN: 1432-1173
- BARAN R ET AL: "Combination of fluconazole and urea in a nail lacquer for treating onychomycosis" JOURNAL OF DERMATOLOGICAL TREATMENT 200502 NO, vol. 16, no. 1, February 2005 (2005-02), pages 52-55, XP009119926 ISSN: 0954-6634
- BARAN R ET AL: "Chemical avulsion with urea nail lacquer" JOURNAL OF DERMATOLOGICAL TREATMENT 200212 GB, vol. 13, no. 4, December 2002 (2002-12), pages 161-164, XP009119925 ISSN: 0954-6634
- SUDAXSHINA MURDAN: "Enhancing the nail permeability of topically applied drugs" EXPERT OPINION ON DRUG DELIVERY, ASHLEY PUBLICATIONS, vol. 5, no. 11, 1 November 2008 (2008-11-01), pages 1267-1282, XP009110859 ISSN: 1742-5247

## Description

The present invention relates to the treatment of hyperkeratosis of pathological nails.

Many diseases affect nails and bother many individuals.

Among them, mention may be made of nail psoriasis and onychomycoses.

Psoriasis is a chronic disease which causes significant suffering and morbidity. The nail is affected in 10 to 50% of the cases (Scher, 1985; van Laborde and Scher, 2000), and it is estimated that 80 to 90% of psoriatic patients have nails affected at a moment of their life (De Berker, 2000). Ungual psoriasis in the absence of skin disease accounts for 1 to 5% of the patients (van Laborde and Scher, 2000).

Ungual psoriasis is painful and invalidating. More than 50% of the patients suffer from nail changes due to ungual psoriasis, about 60% estimate that ungual psoriasis limits their daily activities and 93% consider ungual psoriasis as a cosmetic handicap (De Jong *et al.,* 1996).

Fingernails are more frequently affected than toenails.

Treatment of ungual psoriasis is focused on improving functional and psychosocial aspects of the disease since no curative treatment exists today.

In spite of recent advances in the treatment of skin psoriasis, the treatment options for ungual psoriasis are much more limited. Depending on the disease, a distinction is made between topic, systemic and intralesional injection corticotherapies, or even PUVA therapy. Systemic therapy has the drawbacks of systemic secondary effects and drug interactions (Murdan, 2002). Intralesional injections of corticosteroids, have shown their effectiveness in certain cases of ungual matrix psoriasis but are extremely painful and have local secondary effects. Topical therapy is the primary therapy for skin psoriasis. However its use on nails is not very documented and proof of its effectiveness on nail dystrophies is an extrapolation of the considered advantages for treating lesions of the skin. In order to be effective, the drug should penetrate through the plate of the nail in order to reach the bed of the nail and the matrix target. Unfortunately, topical drugs formulated for treating skin diseases are not adapted for optimizing the penetration of drugs in and through the plate of the nail, and diffusion in the strongly keratinized plate of the nail of available formulations, regardless of the active substance (for example corticosteroids, calcipotriol, 5-fluorouracil), is very low. Topical treatment of ungual psoriasis by using commercial available formulations is therefore disappointing.

Nail varnish compositions comprising urea have been disclosed e.g. in the patent documents FR2613227, WO2005/011565 and WO02/098380.

An onychomycosis is defined as a fungic infection of the ungual system, i.e. of the matrix, the bed or the plate of the nail caused by dermatophytes (of the genus *Trichophyton*, *Epidermophyton* or *Microsporum*), yeasts (*Candida* or *Malassezia)* or fungi (*Fusarium*). At the fingers, these are most often yeasts (*Candida*).

Onychomycosis is the most frequent of ungual pathologies. It concerns 6 to 9% of the general population.

This is especially a disease of adults; it is rare in children. Its prevalence increases with age; it is 30% after 70 years of age.

90% of the onychomycoses affect the toes and in 9 cases out of 10, these are dermatophytes.

Onychomycosis never cures spontaneously and is known as being difficult to treat.

Ungual hyperkeratosis is one of the symptoms of ungual psoriasis and of onychomycoses. This is an excessive proliferation of the bed of the nail which may lead to onycholysis. It results from the deposition of cells under the plate of the nail which have not been eliminated by desquamation. As this is a symptom which only affects the bed of the nail, it may be treated with a topical product such as varnish. Hyperkeratosis may be painful, may reduce the penetration of topical drugs and is not aesthetic.

As compared with creams and ointments, curative nail varnishes are relatively new formulations, known as transungual release devices (Murdan, 2002). The varnish forms a film which adheres to the plate of the nail and does not flake off during daily activities. The film acts as a drug reservoir from where the drug is released, penetrating and acting in the nail during the whole duration of the application. Further, the formation of a film on the plate of the nail reduces the water loss of the surface of the nail, resulting in hyperhydration of the upper layers of the nail, which may also contribute to diffusion of the drug.

Urea has been used for long time in dermatology for its keratolytic properties.

There is a significant need for a nail varnish comprising a high proportion of urea intended for treating ungual hyperkeratosis providing the patient with improved comfort by reducing the thickness of the plate of the nail and by improving the aesthetic aspect of the nail as well as better compliance with the treatment, considering its ease of application.

The problem however is the following: for significant amounts of urea the solution precipitates before and/or after application.

There is a need for a film-forming transparent solution containing a high proportion of urea and intended for treating ungual hyperkeratosis.

Surprisingly, the inventors have developed a nail varnish, the composition of which provides urea amounts of 15% and transparence in the flask and after application.

The object of the present invention is thus a film-forming solution comprising:
- 10 to 20 % of urea,
- 5 to 15 % of film-forming polymer,
- 45 to 65 % of a polar solvent,
- 1 to 20 % of a co-solvent,
- 0.01 to 5%, for example 0.01% to 1%, for example 0.5 to 5%, for example from 0.5 to 1% of a plasticizer or a second co-solvent and
- water up to 100%.

Preferentially, the film-forming solution according to the invention comprises 13-17% of urea. More preferably, the film-forming solution according to the invention comprises 17% of urea.

Preferentially, the film-forming solution according to the invention comprises 8 to 12% of a film-forming polymer. More preferably, the film-forming solution according to the invention comprises 10% of a film-forming polymer.

Preferentially, the film-forming solution according to the invention comprises 45 to 50% of a polar solvent. More preferably, the film-forming solution according to the invention comprises 48 to 49% of a polar solvent.

Preferentially, the film-forming solution according to the invention comprises 1 to 5% of a co-solvent. More preferably, the film-forming solution according to the invention comprises 4 to 5% of a co-solvent.

Preferentially, the film-forming solution according to the invention comprises 0.5 to 1% of a plasticizer. More preferably, the film-forming solution according to the invention comprises 0.6 to 0.7% of a plasticizer.

Advantageously, the film-forming polymer of the film-forming solution according to the invention is a Eudragit. Preferably, the film-forming polymer of the film-forming solution according to the invention is a Eudragit E, RL, RS, L or S. More preferably, the film-forming polymer of the film-forming solution according to the invention is Eudragit E100, Eudragit RL/RS, Eudragit L100, Eudragit S100 and Eudragit L100-55.

In the sense of the present invention, a polymer of methacrylates and/or acrylates is called a «Eudragit ».

Eudragit E100 is a copolymer of methyl methacrylate and of butyl methacrylate.

Eudragit RL/RS is a copolymer of trimethyl ammonioethyl methacrylate chloride.

Eudragit L are copolymers of methacrylic acid and of ethyl acrylates.

Eudragit S are copolymers of methacrylic acid and of methacrylates.

Advantageously, the polar solvent of the film-forming solution according to the invention is ethyl alcohol, preferably 96% ethyl alcohol.

Advantageously, the co-solvent of the film-forming solution according to the invention is selected from the list consisting of propylene glycol, glycerin, sorbitol, ethoxyglycol, ethyl acetate, isopropanol, butyl alcohol, and polyethylene glycol 200, for example from the list consisting of propylene glycol, glycerin, sorbitol and polyethylene glycol 200. Indeed, these co-solvents have sufficient volatility in order to achieve rapid drying of the varnish while improving the solubility of the urea in the presence of the film-forming polymer. Preferably, the co-solvent of the film-forming solution according to the invention is propylene glycol which has proved to be the best co-solvent for solubilizing urea in the presence of the film-forming polymer.

Advantageously, the plasticizer or the second co-solvent of the film-forming solution according to the invention is selected from the list consisting of diethyl phthalate, triethyl citrate, triacetine, dibutyl sebacate, diethyl sebacate, dibutyl phthalate, acetyltriethyl citrate, and polyethylene glycols. Indeed, with these plasticizers and co-solvents it is possible to obtain the sought transparency. For example, the plasticizer or the second co-solvent of the film-forming solution according to the invention is selected from the group consisting of diethyl phthalate, triethyl citrate, dibutyl sebacate, diethyl sebacate, dibutyl phthalate, acetyltriethyl citrate, and polyethylene glycols. Diethyl phthalate further has an interesting potential as a bitterness agent which may be prove to be useful in a nail varnish for nail-biting patients. According to an embodiment of the invention, the mentioned plasticizers are used as an additional co-solvent, for example the solution according to the invention comprises from 0.01 to 5%, for example from 0.01% to 1%, for example from 0.5 to 5%, for example from 0.5 to 1% of said agents in addition to the co-solvents mentioned earlier.

In an embodiment of the invention, the film-forming solution comprises 10 to 20% of urea, 45 to 65% of a polar solvent, 5 to 15% of a co-solvent, 5 to 15% of a film-forming agent, 0.5 to 5%, for example 0.5 to 1% of a second so-solvent and water up to 100%.

In an embodiment of the invention, the film-forming solution comprises 10 to 20% of urea, 45 to 65% of 96% ethyl alcohol, 5 to 15% of propylene glycol, 5 to 15% of Eudragit E100, 0.5 to 5% for example 0.5 to 1% of diethyl phthalate and water up to 100%.

A preferred film-forming solution of the invention comprises:
- 15 % of urea,
- 48.69 % of 96% ethyl alcohol,
- 4.4 % of propylene glycol,
- 10 % of Eudragit E100,
- 0.63 % of diethyl phthalate and
- 21.28 % of water.

Advantageously, the film-forming solution according to the invention is used for treating nails having hyperkeratosis. This hyperkeratosis may be related to another pathology, for example a hyperkeratosis related to lupus..

Another object of the invention is a film-forming solution according to the invention for a use intended to remove abnormal ungual keratinous material, in particular abnormal ungual keratinous material resulting from psoriasis or onychomycosis.

Another object of the invention is the use of a film-forming solution according to the invention for making a drug intended for removing abnormal ungual keratinous material.

Preferably, this abnormal ungual keratinous material results from onychomycosis or psoriasis.

The following examples illustrate the invention without limiting the scope thereof.

### Example 1: Formulation according to the invention

### Formula SR2852

| Material designation | Amounts g/100 g |
|---|---|
| Urea | 15.00 |
| 96% ethyl alcohol | 48.69 |
| Propylene glycol | 4.40 |
| Eudragit E100 | 10.00 |
| Diethyl phthalate | 0.63 |
| Purified water | 21.28 |

After applying the formula SR2852, a transparent film is formed on the nail. The urea does not crystallize after drying of the varnish on the nail.

### Example 2: Formulation without any plasticizer (comparative test)

### Formula SR2893

| Material designation | Amounts g/100 g |
|---|---|
| Urea | 15.00 |
| 96% ethyl alcohol | 49.32 |
| Propylene glycol | 4.40 |
| Eudragit E100 | 10.00 |
| Purified water | 21.28 |

After applying the formula SR2893, an opaque film is formed on the nail. The film is the result of recrystallization of urea after drying the varnish on the nail

### Example 3: Method for preparing a formulation according to the invention

The operating procedure for making the formula or Example 1 for a batch of 500 g is the following:
In a beaker 106.4 g of water and 75 g of urea are introduced with stirring. To the solution are added 22 g of propylene glycol, 243.45 g of ethyl alcohol, and 3.15 g of diethyl phthalate and 50 g of Eudragit E100. Stirring is performed until the solution becomes limpid.

### Example 4: Effectiveness and tolerance of the medical device with 15% urea nail varnish in the treatment of hyperkeratosis of psoriatic nails of the hand: an exploratory study.

1- Main goal: evaluate the effectiveness of a nail varnish with 15% of urea for treating hyperkeratosis of psoriatic nails of the hand after 6 months of daily application.

### 2- Secondary goals:

evaluation of the success of the treatment after 6 months
global self-evaluation of the treatment by the patient after 6 months
evaluate local and general tolerance of the product
evaluation of the life quality of the patient

### 3- Composition of the medical device: urea 15%, ethanol, propylene glycol, Eudragit E100, diethyl phthalate, purified water.

Administration: the product is applied once daily just before going to bed for 6 months.

### 4 - Inclusion criteria

- man or woman of more than 18 years of age
- patient with a history of skin psoriasis
- patient having at least two nails with hyperkeratosis with a thickness of more than 2 mm

### 5 - Evaluation criterion:

### 5.1- Main criterion:

Effectiveness of the nail varnish with 15% of urea for treating hyperkeratosis of psoriatic nails after 6 months.

For each patient, the thickness of the treated nail is measured in millimeters with a vernier caliper. At each visit, the measurement is made at the same nail area (defined as the initial condition), where the thickness is the largest at the moment of the inclusion.

### 5.2 - Intermediate secondary criteria:

- measurement of hyperkeratosis after 1.5 and 3 months.
- the investigator determines a global dynamic score (dynamic Investigator Physician Global Assessment) which assesses global improvement after 1.5, 3 and 6 months as compared with the initial condition. At each visit, a global clinical assessment is carried out by comparison with photographs taken at the inclusion.
- the investigator determines a global statistic score (static Investigator Physician Global Assessment, sIPGA) at each visit (inclusion, after 1.5, 3 and 6 months) without any comparison with the initial condition. The severity of psoriasis, i.e. the ungual surface having psoriatic lesions, is assessed according to the scale detailed below. The affected nails are not assessed separately, but a global score is assigned for the whole of the affected nails.

### 6 - Conclusion

The provided results correspond to intermediate results after 3 months of application.

28 patients were analyzed for these intermediate results, 4 patients were withdrawn from the study.

As regards effectiveness, a significant reduction in hyperkeratosis was observed after 3 months of treatment. This reduction is of -25% after 3 months and was -10 % after 1.5 months of treatment. A daily application of 15% nail varnish containing 15% of urea for treating psoriasis of the nail shows a significant effect in the reduction of hyperkeratosis, and trends in favor of the effectiveness for the other criteria (global dynamic score, global static score). Good tolerance is observed.

## Claims

1. A film-forming solution comprising:
- 10 to 20 % of urea,
- 5 to 15 % of a film-forming polymer,
- 45 to 65 % of a polar solvent,
- 1 to 20 % of a co-solvent,
- 0.01 to 5% of a plasticizer selected from the list consisting of diethyl phthalate, triethyl citrate, dibutyl sebacate, diethyl sebacate, dibutyl phthalate, acetyltriethyl citrate, and polyethylene glycols, and
- water up to 100%.

2. The film-forming solution according to claim 1, comprising 13 to 17% of urea.

3. The film-forming solution according to claim 1 or 2, comprising 15% of urea.

4. The film-forming solution according to any of the preceding claims, comprising :
- 8 to 12 % of a film-forming polymer,
- 45 to 50 % of a polar solvent,
- 1 to 5 % of a co-solvent,
- 0.5 to 1% of a plasticizer and
- water up to 100%.

5. The film-forming solution according to any of the preceding claims, the film-forming polymer being a Eudragit.

6. The film-forming solution according to claim 5, the film-forming polymer being selected from the list consisting of Eudragit E100, Eudragit RL/RS, Eudragit L100, Eudragit S100 and Eudragit L100-55.

7. The film-forming solution according to any of the preceding claims, the polar solvent being ethyl alcohol.

8. The film-forming solution according to any of the preceding claims, the co-solvent being selected from the list consisting of propylene glycol, glycerin, sorbitol and polyethylene glycol 200.

9. The film-forming solution according to claim 1, comprising 10 to 20% of urea, 45 to 65% of 96% ethyl alcohol, 5 to 15% of propylene glycol, 5 to 15% of Eudragit E100, 0.5 to 1% of diethyl phthalate and water up to 100%.

10. The film-forming solution according to any of claims 1 to 8, comprising:
- 15 % of urea,
- 48.69 % of 96% ethyl alcohol,
- 4.4 % of propylene glycol,
- 10 % of Eudragit E100,
- 0.63 % of diethyl phthalate and
- 21.28 % of water.

11. The film-forming solution according to any of the preceding claims, for a use intended for removing abnormal ungual keratinous material.

12. The film-forming solution for the use according to claim 11, the abnormal ungual keratinous material resulting from psoriasis or onychomycosis.

## Patentansprüche

1. Eine filmbildende Lösung, umfassend:
- 10 bis 20 % Harnstoff,
- 5 bis 15 % eines filmbildenden Polymers,
- 45 bis 65 % eines polaren Lösungsmittels,
- 1 bis 20 % eines Co-Lösungsmittels,
- 0,01 bis 5 % eines Weichmachers, der aus der Liste bestehend auf Diethyl-Phthalat, Triethyl-Citrat, Dibutyl-Sebacat, Diethyl-Sebacat, Dibutyl-Phthalat, Acetyltriethyl-Citrat und Polyethylen-Glykolen ausgewählt ist und
- Wasser bis zu 100 %.

2. Die filmbildende Lösung nach Anspruch 1, umfassend 13 bis 17 % Harnstoff.

3. Die filmbildende Lösung nach Anspruch 1 oder 2, umfassend 15 % Harnstoff.

4. Die filmbildende Lösung nach einem der voranstehenden Ansprüche, umfassend:
- 8 bis 12 % eines filmbildenden Polymers,
- 45 bis 50 % eines polaren Lösungsmittels,
- 1 bis 5 % eines Co-Lösungsmittels,
- 0,5 bis 1 % eines Weichmachers und
- Wasser bis zu 100 %.

5. Die filmbildende Lösung nach einem der voranstehenden Ansprüche, wobei das filmbildende Polymer ein Eudragit ist.

6. Die filmbildende Lösung nach Anspruch 5, wobei das filmbildende Polymer aus der Liste bestehend ein Eudragit E100, Eudragit RL/RS, Eudragit L100, Eudragit S100 und Eudragit L100-55 ausgewählt ist.

7. Die filmbildende Lösung nach einem der voranstehenden Ansprüche, wobei das polare Lösungsmittel Ethylalkohol ist.

8. Die filmbildende Lösung nach einem der voranstehenden Ansprüche, wobei das Co-Lösungsmittel aus der Liste bestehend aus Propylen-Glykol, Glycerin, Sorbitol und Polyethylen-Glykol 200 ausgewählt ist.

9. Die filmbildende Lösung nach Anspruch 1, umfassend 10 bis 20 % Harnstoff, 45 bis 65 % Ethylalkohol 96 %, 5 bis 15 % Propylen-Glykol, 5 bis 15 % Eudragit E100, 0,5 bis 1 % Diethyl-Phthalat und Wasser bis zu 100 %.

10. Die filmbildende Lösung nach Anspruch 1 bis 8, umfassend:
- 15 % Harnstoff,
- 48,69 % Ethylalkohol 96 %,
- 4,4 % Propylen-Glykol,
- 10 % Eudragit E100,
- 0,63 % Diethyl-Phthalat und
- 21,28 % Wasser.

11. Die filmbildende Lösung nach einem der voranstehenden Ansprüche für die Verwendung zur Entfernung von abnormalem, ungualem, keratinösem Material.

12. Die filmbildende Lösung für die Verwendung nach Anspruch 11 von abnormalem ungualem keratinösem Material, das aus Psoriasis oder Onychomycosis resultiert.

## Revendications

1. Solution filmogène comprenant :
- 10 à 20 % d'urée,
- 5 à 15 % d'un polymère filmogène,
- 45 à 65 % d'un solvant polaire,
- 1 à 20 % d'un co-solvant,
- 0,01 à 5% d'un agent plastifiant choisi dans la liste constituée du phtalate de diéthyle, du citrate de triéthyle, du sébacate de dibutyle, du sébacate de diéthyle, du phtalate de dibutyle, de citrate d'acétyle et de triéthyle et des polyéthylène glycols, et
- jusqu'à 100% d'eau.

2. Solution filmogène selon la revendication 1, comprenant 13 à 17 % d'urée.

3. Solution filmogène selon la revendication 1 ou 2, comprenant 15 % d'urée.

4. Solution filmogène selon l'une quelconque des revendications précédentes, comprenant :
- 8 à 12 % d'un polymère filmogène,
- 45 à 50 % d'un solvant polaire,
- 1 à 5 % d'un co-solvant,
- 0,5 à 1% d'un agent plastifiant et
- jusqu'à 100% d'eau.

5. Solution filmogène selon l'une quelconque des revendications précédentes, le polymère filmogène étant un Eudragit.

6. Solution filmogène selon la revendication 5, le polymère filmogène étant choisi dans la liste constituée de l'Eudragit E100, l'Eudragit RL/RS, l'Eudragit L100, l'Eudragit S100 et l'Eudragit L100-55.

7. Solution filmogène selon l'une quelconque des revendications précédentes, le solvant polaire étant l'alcool éthylique.

8. Solution filmogène selon l'une quelconque des revendications précédentes, le co-solvant étant choisi dans la liste constituée du propylène glycol, de la glycérine, du sorbitol et du polyéthylène glycol 200.

9. Solution filmogène selon la revendication 1, comprenant 10 à 20 % d'urée, 45 à 65 % d'alcool éthylique 96 %, 5 à 15 % de propylène glycol, 5 à 15 % d'Eudragit E100, 0,5 à 1 % de phtalate de diéthyle et jusqu'à 100 % d'eau.

10. Solution filmogène selon l'une quelconque des revendications 1 à 8, comprenant :
- 15 % d'urée,
- 48,69 % d'alcool éthylique 96%,
- 4,4 % de propylène glycol,
- 10 % d'Eudragit E100,
- 0,63 % de phtalate de diéthyle et
- 21,28 % d'eau.

11. Solution filmogène selon l'une quelconque des revendications précédentes pour utilisation pour éliminer la matière kératinique unguéale anormale.

12. Solution filmogène pour l'utilisation selon la revendication 11, la matière kératinique unguéale anormale résultant d'un psoriasis ou d'une onychomycose.
